# EUROPEAN PATENT APPLICATION

(11) **EP 4 613 199 A1**
(43) Date of publication of application: **10.09.2025**
(21) Application number: 23885987.0
(22) Date of filing: 05.09.2023
(51) Int. Cl.: A61B 5/318, A61B 5/28, A61B 5/00, G16H 50/20

(54) **METHOD FOR INDUCING ADDITIONAL MEASUREMENT OF ELECTROCARDIOGRAM**

(30) Priority: 02.11.2022 KR 20220144416
(71) Applicant: VUNO INC., Seoul 06541 (KR)
(72) Inventor: JOO, Sunghoon, Seoul 04778 (KR); CHANG, Mineok, Seoul 06655 (KR); LEE, Sungjae, Seoul 04582 (KR)
(74) Representative: Petraz, Gilberto Luigi
(86) International application number: PCT/KR2023/013230
(87) International publication number: WO 2024/096290

(57) **Abstract**

Disclosed is a method performed by a processor for inducing an additional measurement of an electrocardiogram, which may include: obtaining a first type of electrocardiogram signal; and determining whether a second type of electrocardiogram signal different from the first type of electrocardiogram signal is additionally required, based on at least one of information regarding the first type of electrocardiogram signal or diagnostic information based on the first type of electrocardiogram signal.

## Description

### [Technical Field]

The present disclosure relates to a method for inducing an additional measurement of an electrocardiogram signal, and more particularly, to a method for inducing an additional measurement of an additional type of electrocardiogram signal.

### [Background Art]

Electrocardiogram is one of the basic diagnostic methods for heart disease and is an important examination method for diagnosing cardiovascular diseases such as angina, myocardial infarction, and arrhythmia. The heart is a three-dimensional organ, and electrocardiograms have been developed to obtain a lot of information by generally attaching multiple leads or for a long time to identify a pathological condition of this organ.

The electrocardiograms generally include a standard 12-lead electrocardiograph that records signals using 10 electrodes, and a Holter electrocardiograph that continuously records electrocardiograms to observe intermittent abnormal findings such as arrhythmias. In recent years, wearable electrocardiographs (watch-type, patch-type, and pad-type) that improve measurement convenience have been developed, but most of them measure only a single lead signal, and only a few products have a limb lead signal measurement function.

Even if the wearable electrocardiograph has a limb lead function, it is difficult to diagnose a heart disease, which shows clear changes only in the precordial leads, only with a single or limb lead because there is no precordial lead signal as shown in a standard 12-lead electrocardiograph.

Meanwhile, with the rapid development of deep learning technology, various diseases can be detected using only single or limb leads even without precordial lead signals. However, in the case of diseases where changes in precordial leads are important for diagnosis, there is a dissonance between the signals obtained from actual electrocardiographs and the diseases diagnosed with deep learning technology for a user. Therefore, it is necessary to ultimately make the user confirm the precordial lead signals. This calls for the development of an algorithm that can induce the precordial-lead measurements.

### [Disclosure]

### [Technical Problem]

The present disclosure has been made in an effort to provide a technology for inducing measurements of additional types of electrocardiogram signals in addition to basic types of electrocardiogram signals. For example, the present disclosure has been made in an effort to provide a technology that identifies a situation in which a measurement of a precordial lead electrocardiogram (ECG) signal is required in addition to a single limb ECG signal or a limb lead ECG signal, and provides a feedback for such a situation.

**In** order to implement the above-described object, one embodiment of the present disclosure provides a method for inducting an additional measurement of an electrocardiogram, which is performed by a processor in order to induce the additional measurement of the electrocardiogram. The method may include: obtaining a first type of electrocardiogram signal; and determining whether a second type of electrocardiogram signal different from the first type of electrocardiogram signal is additionally required, based on at least one of information regarding the first type of electrocardiogram signal and diagnostic information based on the first type of electrocardiogram signal.

As an alternative embodiment, the first type of electrocardiogram signal may include a single lead electrocardiogram signal or a limb lead electrocardiogram signal.

As an alternative embodiment, the second type of electrocardiogram signal may include a precordial lead electrocardiogram signal.

As an alternative embodiment, the determining of whether the second type of electrocardiogram signal is additionally required may include determining that the second type of electrocardiogram signal is additionally required when the diagnostic information based on the first type of electrocardiogram signal corresponds to a predetermined type of disease.

As an alternative embodiment, the predetermined type of disease may include at least one disease of a disease classified as being more sensitive to the precordial lead than the limb lead, or a disease classified as having different diagnostic schemes for the limb lead and the precordial lead.

As an alternative embodiment, the determining of whether the second type of electrocardiogram signal is additionally required may include determining that the second type of electrocardiogram signal is additionally required when a prediction value based on the first type of electrocardiogram signal is within a predetermined range from a reference value (or a cut-off value).

As an alternative embodiment, the determining of whether the second type of electrocardiogram signal is additionally required may include predicting a difference between first diagnostic information based on the first type of electrocardiogram signal, and second diagnostic information based on the first type of electrocardiogram signal and the second type of electrocardiogram signal, based on the first type of electrocardiogram signal, and determining whether the second type of electrocardiogram signal is additionally required based on the predicted difference.

As an alternative embodiment, the determining of whether the second type of electrocardiogram signal is additionally required may include generating a prediction value by inputting the first type of electrocardiogram signal into a first prediction model having a first reference value (or a first cut-off value), and determining whether the second type of electrocardiogram signal is additionally required based on the generated prediction value, and the second type of electrocardiogram signal may be used to be input into a second prediction model having a second reference value (or a second cut-off value), and the first reference value may be set differently from the second reference value.

As an alternative embodiment, the determining of whether the second type of electrocardiogram signal is additionally required may include determining that the second type of electrocardiogram signal is additionally required when the diagnostic information based on the first type of electrocardiogram signal is generated two times or more within a predetermined period, and an electrocardiogram problem is diagnosed a preset number of times or more by the diagnostic information generated two times or more.

As an alternative embodiment, the determining of whether the second type of electrocardiogram signal is additionally required may include extracting waveforms of a plurality of respective lead signals included in the first type of electrocardiogram signal, and determining whether the second type of electrocardiogram signal is additionally required based on a difference between the waveforms of the plurality of lead signals.

Further, in order to implement the above-described object, another embodiment of the present disclosure provides a computer program stored in a computer-readable storage medium in which when the computer program is executed by one or more processors, the computer program allows the one or more processor to perform operations for inducing an additional measurement of an electrocardiogram, and the operations may include an operation of obtaining a first type of electrocardiogram signal; and an operation of determining whether a second type of electrocardiogram signal different from the first type of electrocardiogram signal is additionally required, based on at least one of information regarding the first type of electrocardiogram signal or diagnostic information based on the first type of electrocardiogram signal.

Further, in order to implement the above-described object, yet another embodiment of the present disclosure provides a computing device for inducing an additional measurement of an electrocardiogram, which includes: a processor including one or more cores; and a memory, in which the processor may obtain a first type of electrocardiogram signal, and determine whether a second type of electrocardiogram signal different from the first type of electrocardiogram signal is additionally required, based on at least one of at least one of information regarding the first type of electrocardiogram signal or diagnostic information based on the first type of electrocardiogram signal.

Further, in order to implement the above-described object, still yet another embodiment of the present disclosure provides a user equipment which includes: at least one processor; and a memory, in which the at least one processor may be configured to determine whether a second type of electrocardiogram signal different from a first type of electrocardiogram signal is additionally required, and generate feedback information related to an additional measurement of the second type of electrocardiogram signal.

Technical solving means which can be obtained in the present disclosure are not limited to the aforementioned solving means and other unmentioned solving means will be clearly understood by those skilled in the art from the following description.

### [Advantageous Effects]

According to one embodiment of the present disclosure, a technology can be provided, which induces measurements for additional types of ECG signals (e.g., a precordial lead ECG signal) in addition to basic types of ECG signals (e.g., a single lead ECG signal or a limb lead ECG signal). Therefore, when additional types of ECG signals are needed to generate more accurate diagnostic information, the present disclosure can generate a feedback for this, thereby generating more accurate diagnostic results.

Meanwhile, the effects of the present disclosure are not limited to the aforementioned effect, and other effects, which are not mentioned above, will be apparent to a person having ordinary skill in the art from the following disclosure.

### [Description of Drawings]

Some of the embodiments are illustrated in the accompanying drawings so as to appreciate features of the present disclosure mentioned as above with reference to the following embodiments through detailed and more concrete description. Further, it is intended that like reference numerals in the drawing denote the same or similar function throughout several aspects. However, the accompanying drawings illustrate only specific typical embodiments of the contents of the present disclosure and are not considered to limit the scope of the present disclosure and it should be noted that other embodiments having the same effect may be sufficiently recognized.
FIG. 1 is a block diagram of a computing device for inducing an additional measurement of an ECG according to one embodiment of the present disclosure.
FIG. 2 is a conceptual diagram illustrating a neural network according to an embodiment of the present disclosure.
FIG. 3 is a flowchart for inducing an additional measurement of an ECG according to one embodiment of the present disclosure.
FIG. 4 is a flowchart for inducing an additional measurement of an ECG based on a type of disease according to one embodiment of the present disclosure.
FIG. 5 is a flowchart for inducing an additional measurement of an ECG based on a predetermined reference value (or a predetermined cut-off value) according to one embodiment of the present disclosure.
FIG. 6 is a flowchart for inducing an additional measurement of an ECG based on a difference between diagnostic information according to one embodiment of the present disclosure.
FIG. 7 is a flowchart for determining whether an ECG signal is additionally required based on quantitative diagnostic information according to one embodiment of the present disclosure.
FIG. 8 is a flowchart for inducing an additional measurement of an ECG based on waveforms for multiple lead signals according to one embodiment of the present disclosure.
FIG. 9 is a simple and normal schematic diagram of an exemplary computing environment in which the embodiments of the present disclosure may be implemented.

### [Best Mode]

Various embodiments will now be described with reference to drawings. In this specification, various descriptions are presented to provide appreciation of the present disclosure. However, it is apparent that the embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

**In** one embodiment of the present disclosure, a computing device may encompass a server and/or a user equipment. The computing device in the present disclosure may include any form of device having computing capabilities. The computing device as a digital device may be a digital device with a calculation capability, which has a processor installed therein and a memory, such as a laptop computer, a notebook computer, a desktop computer, a web pad, or a mobile phone. In one embodiment, when the computing device corresponds to the server, the computing device may be a web server that processes a service. A type of computing device described above is just an example and the present disclosure is not limited thereto.

Data, as used throughout the detailed description and claims of the present disclosure, may mean material containing any form of value or information, such as an image or text.

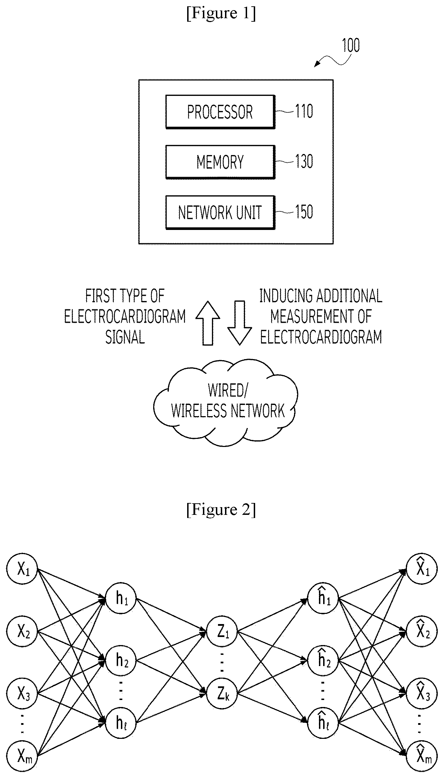

FIG. 1 is a block diagram of a computing device for inducing an additional measurement of an ECG according to one embodiment of the present disclosure.

A configuration of a computing device 100 illustrated in FIG. 1 is only an example shown through simplification. In an embodiment of the present disclosure, the computing device 100 may include other components for performing the computing environment of the computing device 100, and only some of the disclosed components may constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. Further, in an exemplary embodiment of the present disclosure, processors of a plurality of computing devices may be used together to process the learning of the network function and the data classification using the network function. Further, the computer program executed in the computing device according to an exemplary embodiment of the present disclosure may be a CPU, GPGPU, or TPU executable program.

According to an exemplary embodiment of the present disclosure, the memory 130 may store any type of information generated or determined by the processor 110 and any type of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to an exemplary embodiment of the present disclosure may use an arbitrary type known wired/wireless communication systems.

The techniques described herein may be used in other networks in addition to those mentioned above.

According to one embodiment of the present disclosure, the processor 110 may perform an operation to induce a measurement of a second type of electrocardiogram signal in addition to a first type of electrocardiogram signal. Here, the first type of electrocardiogram signal may include a single lead electrocardiogram signal or a limb lead electrocardiogram signal, and the second type of electrocardiogram signal may include a precordial lead electrocardiogram signal.

For example, the processor 110 may ① perform an operation of acquiring the first type of electrocardiogram signal transmitted through the network unit 150, stored in the memory 130, or measured through an independent measurement unit of the computing device 100, and ② perform an operation of determining whether the second type of electrocardiogram signal, which is different from the first type of electrocardiogram signal, is additionally required based on at least one of information about the first type of electrocardiogram signal or diagnostic information based on the first type of electrocardiogram signal. ③ Additionally, when the processor 110 determines that the second type of electrocardiogram signal is additionally required, the processor 110 may generate feedback information to induce an additional measurement of the second type of electrocardiogram signal. Here, the feedback information may be implemented in various forms, including visual information, auditory information, etc. Meanwhile, the computing device 100 may further include a visual output unit such as a display device or an auditory output unit such as a speaker to output such feedback information, and may include various output units to convey the feedback information to the user in addition to such output units. Additionally, the computing device 100 may transmit such feedback information to an external device, such as a smart device, through the network unit 150, and may cause such feedback information to be output through the external device.

FIG. 2 is a schematic diagram illustrating a network function according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

**In** the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

**In** the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

**In** the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

**In** an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data.

That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error.

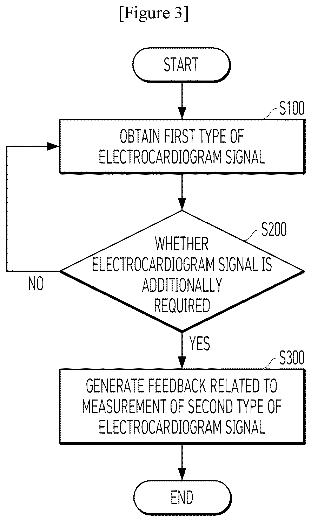

FIG. 3 is a flowchart illustrating a method for inducing an additional measurement of an ECG according to one embodiment of the present disclosure.

Referring to FIG. 3, the method for inducing an additional measurement of an electrocardiogram may be performed by the computing device 100, and may include a step (S100) of obtaining a first type of electrocardiogram signal, a step (S200) of determining whether a second type of electrocardiogram signal, different from the first type of electrocardiogram signal, is additionally required based on at least one of information about the first type of electrocardiogram signal or diagnostic information based on the first type of electrocardiogram signal, and a step (S300) of generating feedback information for inducing a measurement of the second type of electrocardiogram signal when the second type of electrocardiogram signal is additionally required.

An electrocardiogram (ECG) signal is a signal about an action current and an action potential difference according to a contraction of the heart, and may be a type of biosignal induced from a biological signal in the form of an electrical or magnetic signal. The ECG signal may be recorded as a wave curve and is usually displayed as several electrocardiogram curves using an electrocardiograph, and is very important in diagnosing heart abnormalities.

The ECG signal has 10 electrodes and 12 lead signals which are combinations of the electrodes, and the electrodes may include RA, LA, RL, LL, V1, V2, V3, V4, V5, and V6, and the lead signals may include 3 standard limb leads, 3 augmented limb leads, and 6 precordial leads. Additionally, the standard limb leads may include leads I, II, and III, the augmented limb leads may include aVR, aVL, and aVF, and the six precordial leads may include lead signals induced by the precordial electrodes V1, V2, V3, V4, V5, and V6. The lead signal may be used interchangeably as "lead" or "lead ECG signal".

Here, the first type of ECG signal may include a single lead ECG signal or a limb lead ECG signal. The single lead ECG signal may be an ECG signal induced only by two electrodes, mainly measuring lead I, and may be easily measured by a smartphone, a smartwatch, a portable patch, a portable pad, other portable dedicated ECG measuring devices, or a combination thereof. Therefore, in cases of arrhythmias where a single electrocardiogram test may be insufficient for diagnosis, measuring the single lead ECG recorded during daily life may be very useful. Recording the patient's heart rate during activity like this may help diagnose arrhythmia and determine the relationship between the patient's symptoms and arrhythmia, and when using medication, the recorded patient's heart rate may provide data for assessing its effectiveness.

In order to measure the standard limb leads, electrodes may be connected to both arms and one leg (usually a left leg), and the standard limb leads may be measured by including lead I, where the left and right arms are positive and negative, lead II, where one leg and right arm are positive and negative, respectively, and lead III, where one leg and left arm are positive and negative, respectively.

Meanwhile, the second type of electrocardiogram signal may include a precordial lead electrocardiogram signal. Unlike the limb lead, the precordial lead may complement the limb lead by observing a direction and strength of an electrical flow of the heart from a left side.

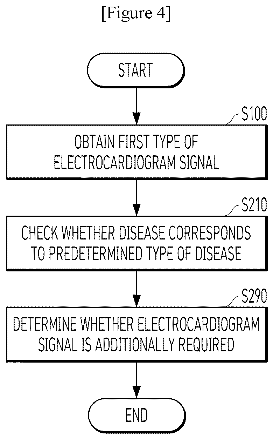

FIG. 4 is a flowchart for inducing an additional measurement of an ECG based on a type of disease according to one embodiment of the present disclosure.

Additionally referring to FIG. 4, the step (S200) of determining whether the second type of electrocardiogram signal is additionally required may include a step (S290) of determining that the second type of electrocardiogram signal is additionally required when the diagnostic information based on the first type of electrocardiogram signal corresponds to a predetermined type of disease (S210). In particular, the predetermined type of disease may be a cardiac abnormality that is difficult to diagnose with a single lead or a limb lead alone.

Here, the predetermined type of disease may include a disease classified as being more sensitive to the precordial lead than to the limb lead. As one example, measuring the precordial lead for an ischemic heart disease may increase a sensitivity and specificity for diagnosis compared to measuring only the limb lead. Therefore, first, the electrocardiogram is monitored only with the first type of electrocardiogram signal that is easily accessible, including the single lead or limb lead, and then, if the ischemic heart disease is suspected, the second type of electrocardiogram signal that includes the precordial lead electrocardiogram is additionally measured, thereby enabling economical and continuous monitoring of the ischemic heart disease and also ensuring the accuracy of diagnosis. Meanwhile, in this case, the step (S200) of determining whether the second type of ECG signal is additionally required may include a step (S290) of checking whether the diagnostic information based on the first type of ECG signal is related to the ischemic heart disease which is one of the predetermined types of diseases, and determining whether the second type of ECG signal is additionally required based on a checking result. Step S290 above may include analyzing, by the processor 110, the diagnostic information based on the first type of ECG signal, and when the ischemic heart disease is suspected, determining that the second type of ECG signal is additionally required.

In addition, the predetermined type of disease may include at least one disease among diseases classified as having different diagnostic schemes for the limb lead and the precordial lead. As an example, left ventricular hypertrophy (LVH) is usually diagnosed by measuring the precordial lead, but may also be diagnosed through the limb lead. Therefore, first, the electrocardiogram is monitored only with the first type of electrocardiogram signal that is easily accessible, including the single lead or limb lead, and then, if the left ventricular hypertrophy (LVH) is suspected, the second type of electrocardiogram signal that includes the precordial lead electrocardiogram is additionally measured, thereby enabling economical and continuous monitoring of the left ventricular hypertrophy (LVH). In addition, the accuracy of the diagnosis may also be secured by cross-validating the diagnosis based on the limb lead and the diagnosis based on the precordial lead. At this time, the diagnosis based on the limb lead or the diagnosis based on the precordial lead may be based on a pre-trained artificial neural network diagnosis model or a rule-based method. Meanwhile, in this case, the step (S200) of determining whether the second type of ECG signal is additionally required may include the step (S290) of checking whether the diagnostic information based on the first type of ECG signal is related to the left ventricular hypertrophy (LVH) which is one of the diseases classified as having different diagnostic schemes, and determining whether the second type of ECG signal is additionally required based on a checking result. The step S290 may include 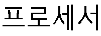 (110) 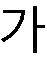 analyzing the diagnostic information based on the first type of ECG signal, and when the left ventricular hypertrophy (LVH) is suspected, determining that the second type of ECG signal is additionally required.

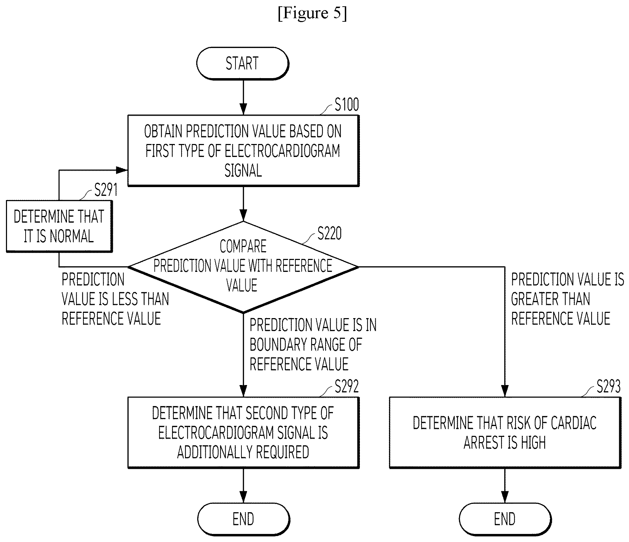

FIG. 5 is a flowchart for inducing an additional measurement of an ECG based on a predetermined reference value (or a predetermined cut-off value) according to one embodiment of the present disclosure.

Additionally referring to FIG. 5, the step (S200) of determining whether the second type of electrocardiogram signal is additionally required may include a step (S292) of determining that the second type of electrocardiogram signal is additionally required when a prediction value based on the first type of electrocardiogram signal is within a predetermined range from a reference value (or a cut-off value). As an example, in a method of measuring the first type of ECG signal from a patient and comparing a prediction value obtained by calculating a risk of heart disease based on the ECG signal with a predetermined reference value (a predetermined cut-off value), waveform information regarding the first type of ECG signal is obtained (S100), and the prediction value obtained by calculating the risk of heart disease based on the waveform information regarding the first type of electrocardiogram signal is set as a first prediction value, and a reference value (a cut-off value) for providing information regarding the risk of heart disease by comparing with the first prediction value is set as a first reference value (or a first cut-off value). At this time, in a conventional case, the information regarding the risk of heart disease may be provided based on comparing the first prediction value with the first reference value, but in the present disclosure, after comparing the first prediction value with the first reference value (S220), if the first prediction value is in a boundary range (or margin range) of the first reference value, it is determined that an additional measurement of the second type of electrocardiogram is required (S292), and a signal may be generated to induce the additional measurement of the second type of electrocardiogram.

Specifically, with respect to the boundary range (or margin range) of the first reference value, if a lowest point of the range is set to a first-first reference value (or a first-first cut-off value) and a highest point of the range is set to a first-second reference value (or a first-second cut-off value), the processor 110 may determine that the risk of heart disease is low, that is, that it is normal if the first reference value is lower than the first-first reference value (S291), and may determine that the second type of electrocardiogram signal is additionally required if the first reference value is equal to or higher than the first-first reference value and lower than the first-second reference value (S292), and may determine that the risk of heart disease (e.g., cardiac arrest) is high if the first reference value is equal to or higher than the first-second reference value (S293). If it is determined that the second type of electrocardiogram signal is additionally required or that the risk of heart disease is high, the processor 110 may deliver, to the user, a feedback signal for additionally inducing a second type of electrocardiogram measurement.

The feedback signal may be delivered to the user as an image, text information, or voice signal through a device directly connected to the computing device 100 or through a network.

Additionally, according to the present disclosure, the user may carry a device capable of measuring the first type of electrocardiogram signal. The device or an application embedded therein may, based on the first type of electrocardiogram signal, deliver data including electrocardiogram information, personal identification information, or current location information of the user to a server of a hospital or rescue organization if it is determined that the second type of electrocardiogram signal is additionally required or that the risk of heart disease is high.

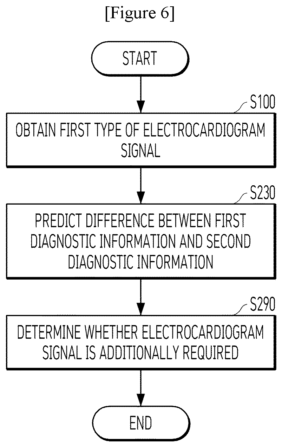

FIG. 6 is a flowchart for inducing an additional measurement of an ECG based on prediction of a difference between diagnostic information according to one embodiment of the present disclosure.

According to one embodiment of the present disclosure, the step (S200) of determining whether the second type of electrocardiogram signal is additionally required may include a step (S230) of predicting a difference between "first diagnostic information" based on the first type of electrocardiogram signal and "second diagnostic information" based on the first type of electrocardiogram signal and the second type of electrocardiogram signal, and a step (S290) of determining whether the second type of electrocardiogram signal is additionally required based on the predicted difference.

Here, step S230 above may be performed by utilizing a neural network model that receives the first type of electrocardiogram signal as an input and predicts a difference between the first diagnostic information and the second diagnostic information. For example, step S230 above may be performed by utilizing a neural network model (binary classification model) that (i) outputs a true prediction value when, after receiving the first type of electrocardiogram signal, the difference between the first diagnostic information and the second diagnostic information is predicted to be large (e.g., when the diagnoses are predicted to be different from each other in a binary diagnosis such as positive/negative, or when the difference between diagnostic values in a quantitative diagnosis is predicted to exceed a preset threshold), and (ii) outputs a false prediction value when the difference between the first diagnostic information and the second diagnostic information is predicted not to be large. Additionally, in the embodiment, the step S290 may include a step of determining that the second type of electrocardiogram signal is additionally required when the true prediction value is generated in the step S230. That is, according to the embodiment, when it is predicted that there will be a large difference between the first diagnostic information (e.g., diagnostic information based on the single lead or limb lead ECG signal) and the second diagnostic information (e.g., diagnostic information based on the 12-lead signal to which the precordial lead ECG signal is added), an additional measurement of the second type of ECG signal (e.g., the precordial lead ECG signal) is induced, thereby preventing erroneous diagnostic information based on the first type of ECG signal (e.g., the single lead ECG signal or the limb lead ECG signal) from being determined.

Meanwhile, the neural network model (binary classification model) that may be utilized in the step S230 may be trained through an exemplary process including: ① an operation of training each of "a first model that predicts diagnostic information based on the first type of electrocardiogram signal" and " a second model that predicts diagnostic information based on the first type of electrocardiogram signal and the second type of electrocardiogram signal"; ② an operation of inputting general electrocardiogram data into the first model and the second model, respectively, to perform prediction; ③ an operation of extracting the corresponding and labeling data as true when prediction values having a large difference are generated by the first model and the second model, and extracting and labeling the corresponding data as false when prediction values having a small difference are generated; ④ an operation of training the neural network model (binary classification model) by utilizing the data labeled in this manner.

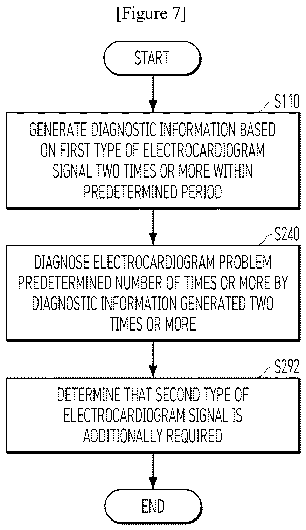

FIG. 7 is a flowchart for determining whether an ECG signal is additionally required based on quantitative ECG analysis information according to one embodiment of the present disclosure.

Referring to FIG. 7, the step (S200) of determining whether the second type of electrocardiogram signal is additionally required may include a step (S290) of determining that the second type of electrocardiogram signal is additionally required when the diagnostic information based on the first type of electrocardiogram signal is generated two times or more within a predetermined period (S110), and ECG problem is diagnosed a preset number of times or more by the diagnostic information generated two times or more (S250).

For example, the processor 110 utilizes a neural network model that provides information for predicting or diagnosing an abnormality in an electrocardiogram based on the first type of electrocardiogram signal (e.g., a single lead electrocardiogram signal or the limb lead electrocardiogram signal), and if, as a result of performing a diagnosis N times (N is a natural number greater than or equal to 2) or more within a predetermined period (e.g., 1 hour, 12 hours, 1 day, etc.), an electrocardiogram problem is diagnosed K times or more (K is a natural number less than N) based on the diagnostic information generated N times, the processor 110 may induce the additional measurement of the second type of electrocardiogram signal in order to induce an additional precise diagnosis.

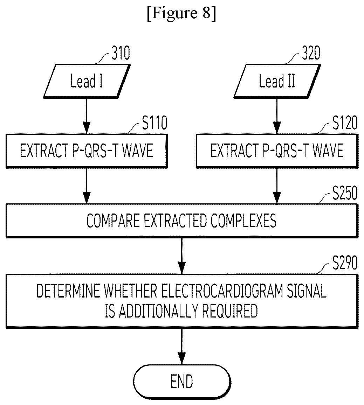

FIG. 8 is a flowchart for inducing an additional measurement of an ECG based on waveforms for multiple lead signals according to some embodiments of the present disclosure.

Referring to FIG. 8, the step (S200) of determining whether the second type of electrocardiogram signal is additionally required includes a step of extracting a waveform of each of multiple lead signals 310 and 320 included in the first type of electrocardiogram signal, and a step (S290) of determining whether the second type of electrocardiogram signal is additionally required based on a difference between the waveforms of the multiple lead signals 310 and 320.

Specifically, when extracting a P-QRS-T waveform for each of lead I (310) and lead II (320) electrocardiograms, if there is a difference in location, shape, or number in one or more complexes among the extracted complexes, an additional measurement is presented in the precordial lead to check whether the second type of electrocardiogram signal is additionally required. At this time, the complex may include one or more of an interval of a PR segment, an interval of a QRS segment of the beat, an interval of a QT segment of the beat, or a location of an R-peak of the beat.

As an example, the processor 110 may receive lead I (310) and lead II (320) signals among the limb lead signals included in the first type of electrocardiogram signal, set coordinates of an electrocardiogram graph in each signal, and identify coordinates of p, q, r, s, and t of the beat therein to generate waveform location information of multiple elements. Next, the processor 110 may perform an operation to directly compare waveform location information of lead I with waveform location information of lead II, and if a difference is greater than a preset value, the processor 110 may determine that the second type of electrocardiogram signal is additionally required.

Alternatively, the processor 110 may use the generated waveform location information to derive an interval of a PR segment, an interval of a QRS segment, or an interval value of a QT segment, which are elements included in the lead I (310) and lead II (320) signals, and the processor 110 may determine that the second type of electrocardiogram signal is additionally required when the difference between the values of any one of the interval of the PR segment, the interval of the QRS segment, or the interval of the QT segment of lead I (310) and lead II (320) is greater than or equal to a preset value.

In this way, if it is automatically identified and notified whether the precordial lead is required by comparing lead I (310) and lead II (320) signals of the limb lead signal, the limb lead signal may perform a screening role. As a result, the present disclosure has an effect of reducing a burden on medical staff and reducing a frequency of precordial lead signal measurement required, while enabling accurate monitoring of electrocardiogram signals.

Additionally, according to one embodiment of the present disclosure, the step of determining whether the second type of electrocardiogram signal is additionally required may include ① a step of inputting the first type of electrocardiogram signal (e.g., the single lead electrocardiogram signal or the limb lead electrocardiogram signal) into a first prediction model that predicts cardiovascular abnormalities with a first reference value (or a first cut-off value) to generate a prediction value, and ② a step of determining whether the second type of electrocardiogram signal (e.g., the precordial lead electrocardiogram signal) is additionally required based on the generated prediction value.

At this time, the second type of electrocardiogram signal may be input to a second prediction model (e.g., a prediction model based on the 12-lead signal) that predicts cardiovascular abnormalities with a second reference value (or a second cut-off value), and the second reference value may be set differently from the first reference value.

**In** addition, the first reference value and the second reference value may be set differently from each other in consideration of sensitivity or specificity. For example, the first reference value and the second reference value may be set differently from each other so that a performance of the first prediction model has a higher sensitivity than the second prediction model (e.g., when the sensitivity criterion is 0.8 to 0.9, the first prediction model has a sensitivity of 0.9). When the reference values are set in this manner, the first prediction model may generate alarms with a higher frequency than the second prediction model in relation to cardiovascular abnormalities, and through this operation, the first prediction model may be utilized as a preliminary screening means for diagnosing cardiovascular abnormalities. Meanwhile, one embodiment of the present disclosure may provide a more accurate diagnosis and reduce cases of missing patients by additionally measuring the second type of electrocardiogram signal (e.g., precordial lead electrocardiogram signal) for patients suspected of having a specific disease, even if the first prediction model generates more frequent false alarms.

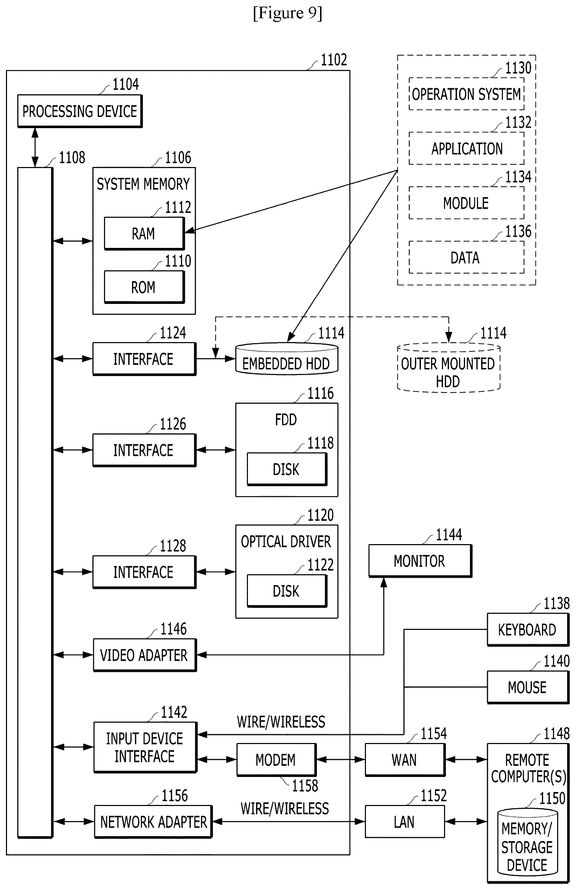

FIG. 9 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

**In** general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multi-processor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

### [Mode for Invention]

Related contents in the best mode for carrying out the present disclosure are described as above.

## Claims

1. A method performed by a processor for inducing an additional measurement of an electrocardiogram, the method comprising:
obtaining a first type of electrocardiogram signal; and
determining whether a second type of electrocardiogram signal different from the first type of electrocardiogram signal is additionally required, based on at least one of information regarding the first type of electrocardiogram signal or diagnostic information based on the first type of electrocardiogram signal.

2. The method of claim 1, wherein the first type of electrocardiogram signal includes a single lead electrocardiogram signal or a limb lead electrocardiogram signal.

3. The method of claim 1, wherein the second type of electrocardiogram signal includes a precordial lead electrocardiogram signal.

4. The method of claim 1, wherein the determining of whether the second type of electrocardiogram signal is additionally required includes:
determining that the second type of electrocardiogram signal is additionally required when the diagnostic information based on the first type of electrocardiogram signal corresponds to a predetermined type of disease.

5. The method of claim 4, wherein the predetermined type of disease includes at least one disease of a disease classified as being more sensitive to a precordial lead than a limb lead, or a disease classified as having different diagnostic schemes for the limb lead and the precordial lead.

6. The method of claim 1, wherein the determining of whether the second type of electrocardiogram signal is additionally required includes:
determining that the second type of electrocardiogram signal is additionally required when a prediction value based on the first type of electrocardiogram signal is within a predetermined range from a reference value.

7. The method of claim 1, wherein the determining of whether the second type of electrocardiogram signal is additionally required includes:
predicting a difference between first diagnostic information based on the first type of electrocardiogram signal, and second diagnostic information based on the first type of electrocardiogram signal and the second type of electrocardiogram signal, based on the first type of electrocardiogram signal, and
determining whether the second type of electrocardiogram signal is additionally required based on the predicted difference.

8. The method of claim 1, wherein the determining of whether the second type of electrocardiogram signal is additionally required includes:
generating a prediction value by inputting the first type of electrocardiogram signal into a first prediction model having a first reference value, and
determining whether the second type of electrocardiogram signal is additionally required based on the generated prediction value,
wherein the second type of electrocardiogram signal is used to be input into a second prediction model having a second reference value, and
wherein the first reference value is set differently from the second reference value.

9. The method of claim 1, wherein the determining of whether the second type of electrocardiogram signal is additionally required includes:
determining that the second type of electrocardiogram signal is additionally required when the diagnostic information based on the first type of electrocardiogram signal is generated two times or more within a predetermined period, and an electrocardiogram problem is diagnosed a predetermined number of times or more by the diagnostic information generated two times or more.

10. The method of claim 1, wherein the determining of whether the second type of electrocardiogram signal is additionally required includes:
extracting a waveform of each of a plurality of lead signals included in the first type of electrocardiogram signal, and
determining whether the second type of electrocardiogram signal is additionally required based on a difference between waveforms of the plurality of lead signals.

11. A computer program stored in a computer-readable storage medium, wherein when the computer program is executed by one or more processors, the computer program allows the one or more processor to perform operations for inducing an additional measurement of an electrocardiogram, and the operations comprising:
an operation of obtaining a first type of electrocardiogram signal; and
an operation of determining whether a second type of electrocardiogram signal different from the first type of electrocardiogram signal is additionally required, based on at least one of information regarding the first type of electrocardiogram signal or diagnostic information based on the first type of electrocardiogram signal.

12. A computing device for inducing an additional measurement of an electrocardiogram, the computing device comprising:
a processor including one or more cores; and
a memory,
wherein the processor is configured to:
obtain a first type of electrocardiogram signal, and
determine whether a second type of electrocardiogram signal different from the first type of electrocardiogram signal is additionally required, based on at least one of information regarding the first type of electrocardiogram signal or diagnostic information based on the first type of electrocardiogram signal.

13. A user equipment comprising:
at least one processor; and
a memory,
wherein the at least one processor is configured to:
determine whether a second type of electrocardiogram signal different from a first type of electrocardiogram signal is additionally required, and
generate feedback information related to an additional measurement of the second type of electrocardiogram signal.
